# FASCICULE DE BREVET EUROPEEN

(11) **EP 2 310 351 B1**
(45) Date de publication et mention de la délivrance du brevet: **09.04.2014**
(21) Numéro de dépôt: 09793949.0
(22) Date de dépôt: 08.07.2009
(51) Int. Cl.: C07C 51/41, C07C 51/48, C07C 53/18, C07C 53/16, C07C 53/19, C07C 53/21

(54) **PROCEDE DE SEPARATION D'UN ACIDE CARBOXYLIQUE SOUS FORME SALIFIEE PORTEUR D'AU MOINS UN ATOME D'HALOGENE.**
VERFAHREN ZUR TRENNUNG EINER CARBONSÄURE IN SALZFORM MIT MINDESTENS EINEM HALOGENATOM
METHOD FOR SEPARATING A CARBOXYLIC ACID IN SALIFIED FORM BEARING AT LEAST ONE HALOGEN ATOM.

(30) Priorité: 10.07.2008 FR 0803932
(43) Date de publication de la demande: 20.04.2011
(73) Titulaire: Rhodia Opérations, 93306 Aubervilliers (FR)
(72) Inventeur: BUISINE, Olivier, F-69230 Saint Genis Laval (FR); METZ, François, F-69540 Irigny (FR)
(74) Mandataire: Dutruc-Rosset, Marie-Claude
(86) Numéro de dépôt international: PCT/EP2009/058688
(87) Numéro de publication internationale: WO 2010/003986

(56) Documents cités:
- US-B1- 6 281 374

## Description

La présente invention a pour objet un procédé de séparation d'un acide carboxylique sous forme salifiée porteur d'au moins un atome d'halogène en position α du groupe carbonyle à partir d'un milieu le comprenant.

Plus précisément, l'invention se rapporte à la séparation d'un acide carboxylique sous forme salifiée porteur d'au moins un atome de fluor en position α du groupe carbonyle.

L'invention vise plus particulièrement la séparation des acides difluoro- et perfluorocarboxyliques sous forme salifiée à partir des milieux de synthèse les comprenant.

Il existe différents modes de préparation des acides difluoro- et perfluorocarboxyliques.

Les dérivés fluorés de nature aliphatique, c'est-à-dire les dérivés fluorés dans lesquels le fluor se trouve porté, au moins en partie, par un carbone sp³, sont en général obtenus par un échange d'un atome d'halogène par un atome de fluor. Cet échange se fait en général en utilisant l'acide fluorhydrique ou bien des sels de l'acide fluorhydrique.

Un autre procédé de préparation décrit dans EP-A 1137615 réside dans un procédé d'hydrogénodéhalogénation sélective qui consiste à substituer un atome d'halogène plus lourd que le fluor par un atome d'hydrogène. US 6 28 13 74 décrit un procédé de séparation d'acides carboxyliques perfluorés.

Ce procédé se définit en ce qu'il comporte une étape d'hydrogénation d'un substrat comportant un atome de carbone d'hybridation sp³ porteur d'un groupe électro-attracteur et d'au moins un atome de fluor et d'un atome d'halogène plus lourd que le fluor, en milieu aqueux basique et en présence d'un métal du groupe VIII, de préférence le nickel de Raney.

A titre d'exemples, on précise que le difluoroacétate de sodium est obtenu avec un excellent rendement supérieur à 90 %, par hydrogénation sous pression, de l'acide chlorodifluoroacétique, en solution sodique et en présence de nickel de Raney.

Ainsi, en fin de réaction, on obtient un milieu aqueux comprenant le sel de sodium de l'acide difluoroacétique, du chlorure de sodium formé au cours de la réaction et un excès de soude.

Le problème qui se pose est qu'il est difficile de séparer le sel de sodium de l'acide difluoroacétique formé au cours de la réaction car c'est un produit soluble dans l'eau et qui est en présence d'autres sels dans le milieu ce qui rend la séparation par filtration impossible.

Généralement, la technique classique utilisée par l'homme du métier est de séparer ledit acide par une acidification suivie d'une extraction ce qui implique d'avoir recours à un solvant organique dans lequel ledit acide soit soluble. La difficulté est d'identifier des solvants permettant la solubilisation et l'extraction de l'acide à partir d'une phase aqueuse.

Pour pallier, cet inconvénient, la présente invention propose un procédé permettant de remédier aux inconvénients précités.

Il a maintenant été trouvé et c'est ce qui constitue l'objet de la présente invention, un procédé de séparation d'un acide carboxylique sous forme salifiée porteur d'au moins un atome d'halogène en position α du groupe carbonyle à partir d'un milieu aqueux le comprenant, caractérisé par le fait que l'on met en contact ce dernier avec un sel d'onium conduisant à la formation de deux phases :
- une phase organique comprenant le sel résultant de la réaction du sel de l'acide carboxylique porteur d'au moins un atome d'halogène en position α du groupe carbonyle et du sel d'onium conduisant au déplacement du cation de l'acide carboxylique par l'onium,
- une phase aqueuse comprenant les différents sels en particulier celui résultant de la réaction du cation de l'acide carboxylique avec l'anion de l'onium,
et par le fait que l'on sépare ensuite les phases organique et aqueuse et que l'on récupère le sel d'onium de l'acide carboxylique à partir de la phase organique.

Dans le présent texte, le sel d'onium de l'acide carboxylique est également dénommé par le terme « complexe ».

Selon une variante du procédé de l'invention, la phase organique peut être diluée avec un solvant dans lequel elle est soluble et l'on récupère le sel d'onium de l'acide carboxylique à partir de ladite phase organique.

Afin d'illustrer le procédé de l'invention et sans aucun caractère limitatif, la Demanderesse cite le cas du sel de sodium de l'acide difluoroacétique qui est obtenu comme précédemment mentionné dans un milieu comprenant du chlorure de sodium et un excès de soude.

Conformément au procédé de l'invention, on met en contact le milieu obtenu avec un sel d'onium et plus préférentiellement avec l'hydrogénosulfate de tétra-n-butylammonium conduisant à la réaction suivante :

On donne ci-après, le schéma réactionnel du procédé de l'invention pour faciliter la compréhension de l'invention sans pour autant lier la portée de l'invention, à celui-ci.

H-CF₂-COONa + n Bu₄-N⁺ HSO₄⁻→ H-CF₂-COO^{- +}N-n Bu₄ + NaHSO₄ + H₂O

La phase organique comprenant H-CF₂-COO- ⁺N-n Bu₄ peut être séparée de la phase aqueuse.

L'invention s'applique à tout composé organique acide comprenant au moins un anion carboxylate présent dans un milieu aqueux, en présence d'autres sels résultant de son procédé de préparation.

Ainsi, l'invention vise aussi bien les monoacides, les diacides ou des mélanges d'acides.

Les substrats plus particulièrement concernés par la présente invention répondent à la formule suivante : 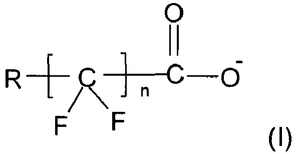 dans ladite formule :
- n est un nombre compris entre 0 et 10,
- si n est égal à 0, R représente un groupe R₁R₂CF de formule (II) dans lequel R₁ et R₂ représentent un atome d'hydrogène, un atome de fluor, chlore ou brome,
- si n est différent de 0, R représente un atome d'hydrogène, un atome de fluor, chlore ou brome ou une fonction carboxylate.

Les substrats mis en oeuvre préférentiellement répondent à la formule (I) dans laquelle n est égal à 0 et R représente un groupe de formule (II).

Encore plus préférentiellement, les substrats répondent à la formule (I) dans laquelle n est égal à 0 et R représente un groupe R₁R₂CF de formule (II) dans lequel R₁ et R₂ représentent un atome d'hydrogène et/ou un atome de fluor.

Comme exemples de substrats, on peut citer les sels des acides mono-et dicarboxyliques, seuls ou en mélange :
- l'acide fluoroacétique,
- l'acide difluoroacétique, DFA
- l'acide trifluoroacétique, TFA
- l'acide chlorodifluoroacétique, CDFA
- l'acide bromodifluoroacétique,
- l'acide perfluoropropanoïque,
- l'acide perfluorobutanoïque,
- l'acide perfluoroheptanoïque,
- l'acide perfluorooctanoïque
- l'acide perfluorodécanoïque,
- l'acide tétrafluorosuccinique,
- l'acide hexafluoroglutarique.

L'invention s'applique plus particulièrement aux sels des acides DFA ou TFA ou à leurs mélanges.

Dans le procédé de l'invention, les acides carboxyliques sont sous forme salifiée, de préférence sous forme d'un sel de métal alcalin, préférentiellement le sodium ou le potassium

Conformément au procédé de l'invention, on met en contact, le sel dudit ou desdits acides carboxyliques, en présence d'un sel d'onium.

Les sels d'oniums susceptibles d'être utilisés dans le procédé de l'invention sont ceux dont les ions onium dérivent notamment de l'azote, du phosphore, du soufre, de l'oxygène, du carbone ou de l'iode et coordiné à des restes hydrocarbonés. Les ions onium dérivant de l'azote ou du phosphore seront quadricoordinés, les ions onium dérivant du soufre, de l'oxygène, du carbone ou de S=O seront tricoordinés tandis que les ions onium dérivant de l'iode seront dicoordinés.

Les restes hydrocarbonés coordinés à ces différents éléments sont des groupes alkyles, alcényles, aryles, cycloalkyles, aralkyles éventuellement substitués, deux restes hydrocarbonés coordinés pouvant former ensemble un groupe unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581 - 587 (1968), les termes "dure" et "intermédiaire" ayant respectivement la signification des termes de "hard" et "borderline" utilisés dans cette référence.

Parmi les ions onium pouvant être utilisés dans le présent procédé de l'invention, conviennent particulièrement ceux répondant à l'une des formules générales suivantes : 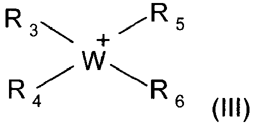 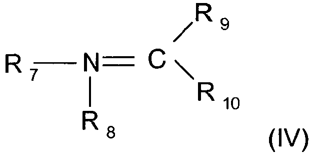 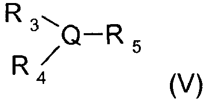 dans lesdites formules :
- W représente N ou P,
- Q représente S, S=O ou C,
- R₃, R₄, R₅ et R₆, identiques ou différents représentent :
   - un groupe alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
   - un groupe alcényle, linéaire ou ramifié, ayant 2 à 12 atomes de carbone ;
   - un groupe aryle ayant 6 à 10 atomes de carbone, éventuellement substitué par un ou plusieurs groupements ou atomes alkyle ayant 1 à 4 atomes de carbone, alkoxy, alkoxycarbonyle, le groupe alkoxy ayant 1 à 4 atomes de carbone, ou halogène ;
   - deux desdits groupes R₃ à R₆ pouvant former ensemble un groupe alkylène, alcénylène ou alcadiènylène, linéaire ou ramifié ayant de 3 à 6 atomes de carbone ;
- R₇, R₈, R₉, R₁₀ sont identiques ou différents et représentent :
   - un atome d'hydrogène,
   - un groupe alkyle linéaire ou ramifié contenant de 1 à 6 atomes de carbone ;
   - les groupes R₉ et R₁₀ pouvant former ensemble un groupe alkylène contenant de 3 à 6 atomes de carbone ;
   - les groupes R₈ et R₉ ou R₈ et R₁₀ pouvant former ensemble un groupe alkylène, alcénylène ou alcadiènylène, contenant 3 ou 4 atomes de carbone et constituant avec l'atome d'azote un hétérocycle azoté éventuellement substitué comme précité et éventuellement l'un des atomes de carbone pouvant être remplacé par un atome d'azote éventuellement porteur d'un groupe R₁₁ qui est un groupe alkyle linéaire ou ramifié contenant de 1 à 20 atomes de carbone.

Parmi les oniums de formule (III) ceux qui sont préférés répondent à la formule (III) dans laquelle W est un atome d'azote ou de phosphore et R₃, R₄, R₅ et R₆, identiques ou différents représentent un groupe alkyle, linéaire ou ramifié, ayant 1 à 12 atomes de carbone et un groupe benzyle.

Parmi les oniums de formule (IV), ceux qui sont préférés répondent à l'une des formules suivantes : 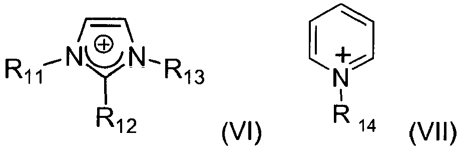 dans ledites formules,
- le groupe R₁₁ représente un groupe alkyle ayant de 1 à 20 atomes de carbone,
- le groupe R₁₂ représente un atome d'hydrogène, un groupe alkyle ayant de 1 à 4 atomes de carbone,
- le groupe R₁₃ représente un groupe alkyle ayant de 1 à 4 atomes de carbone,
- le groupe R₁₄ représente un groupe alkyle ayant de 1 à 6 atomes de carbone.

Parmi les bases "dures" ou "intermédiaires" pouvant constituer l'anion desdits sels d'onium, on peut citer les ions : F^{-,} ClO₄⁻ , PF₆⁻, BF₄⁻, PO₄³⁻, HPO₄^{2-,} H₂PO₄⁻, CH₃SO₃⁻, Ph-SO₃^{-,} CH₃Ph-SO₃⁻, HSO₄⁻, SO₄^{2-,} NO₃⁻, AlCl₄-, Cl^{-,} Br⁻, I⁻, OH⁻, Ph représentant un groupe phényle, ainsi que tous les autres anions répondant à la définition de base "dure" ou "intermédiaire" de PEARSON.

Pour des raisons de commodité de mise en oeuvre, lesdits anions pourront être choisis parmi : bromure, chlorure, hydrogénosulfate ou hydrogénophosphate.

A titre d'exemples d'ions onium répondant à la formule (III), on peut citer les cations :
- tétraméthylammonium,
- triéthylméthylammonium,
- tributylméthylammonium,
- triméthylpropylammonium,
- tétraéthylammonium,
- tétrabutylammonium,
- dodécyltriméthylammonium,
- méthyltrioctylammonium,
- heptyltributylammonium,
- tétrapropylammonium,
- tétrapentylammonium,
- tétrahexylammonium,
- tétraheptylammonium,
- tétraoctylammonium,
- tétradécylammonium,
- butyltripropylammonium,
- méthyltributy4ammonium,
- pentyltributylammonium,
- méthyldiéthylpropylammonium,
- éthyldiméthylpropylammonium,
- tétradodécylammonium,
- tétraoctadécylammonium,
- hexadécyltriméthylammonium,
- benzyltriméthylammonium,
- benzyldiméthylpropylammonium,
- benzyldiméthyloctylammonium,
- benzyltributylammonium,
- benzyltriéthylammonium,
- phényltriméthylammonium,
- benzyldiméthyltétradécylammonium,
- benzyldiméthylhexadécylammonium,
- diméthyldiphénylammonium,
- méthyltriphénylammonium,
- butène-2-yltriéthylammonium,
- N,N-diméthyl-tétraméthylèneammonium,
- N;N-diéthyl-tétraméthylèneammonium,
- tétraméthylphosphonium,
- tétrabutylphosphonium,
- éthyltriméthylphosphonium,
- triméthylpentylphosphonium,
- octyltriméthylphosphonium,
- dodécyltriméthylphosphonium,
- triméthylphénylphosphonium,
- diéthyldiméthylphosphonium,
- dicyclohexyldiméthylphosphonium,
- diméthyldiphénylphosphonium,
- cyclohexyltriméthylphosphonium,
- triéthylméthylphosphonium,
- méthyltri(isopropyl)phosphonium,
- méthyltri(n-propyl)phosphonium,
- méthyltri(isobutyl)phosphonium,
- méthyltri(n-butyl)phosphonium,
- diisobutyl-n-octylméthylphosphonium,
- méthyltri(méthyl-2 propyl)phosphonium,
- méthyltricyclohexylphosphonium,
- méthyltriphénylphosphonium,
- méthyltribenzylphosphonium,
- méthyltri(méthyl-4 phényl) phosphonium,
- méthyltrixylylphosphonium,
- diéthylméthylphénylphosphonium,
- dibenzylméthylphénylphosphonium,
- éthyltriphénylphosphonium,
- tétraéthylphosphonium,
- éthyltri(n-propyl)phosphonium,
- triéthylpentylphosphonium,
- hexadécyltributylphosphonium,
- éthyltriphénylphosphonium,
- n-butyltri(n-propyl)phosphonium,
- butyltriphénylphosphonium,
- benzyltriphénylphosphonium,
- (β-phényléthyl)diméthylphénylphosphonium,
- tétraphénylphosphonium,
- triphényl(méthyl-4 phényl)phosphonium,
- tétrakis(hydroxyméthyl)phosphonium.

Parmi les cations répondant à la formule (IV), on peut citer les cations :
- les 1-alkyl-2,3-diméthylimidazolium,
- les 1-alkyl-3-méthylimidazolium,
- les 1-alkylpyridinium,
- N-méthylpicolinium.

A titre d'exemples d'ions onium répondant à la formule (V), on peut citer les cations :
- triméthylsulfonium,
- triéthylsulfonium,
- triphénylsulfonium,
- triméthylsulfoxonium,
- triphénylcarbénium.

Parmi les ions onium qui peuvent être utilisés dans le cadre du présent procédé, on préférera le plus souvent les ions ammonium quaternaire, les ions phosphonium quaternaire, les ions imidazolinium et pyridinium.

Comme exemples plus spécifiques de sels d'onium, on peut citer les sels de 1-alkyl-2,3-diméthylimidazolium tels que le bromure de 1-éthyl-2,3-diméthylimidazolium, de 1-butyl-2,3-diméthylimidazolium, de 1-hexyl-2,3-diméthylimidazolium ; le chlorure de 1-éthyl-2,3-diméthylimidazolium, de 1-butyl-2,3-diméthylimidazolium, de 1-hexyl-2,3-diméthylimidazolium ; le tétrafluoroborate de 1-butyl-2,3-diméthylimidazolium, de 1-hexyl-2,3-diméthylimidazolium ; les sels de 1-alkyl-3-méthylimidazolium tels que le bromure de 1-éthyl-3-méthylimidazolium, de 1-hexyl-3-méthylimidazolium ; le chlorure de 1-éthyl-3-méthylimidazolium, de 1-butyl-3-méthylimidazolium, de 1-hexyl-3-méthylimidazolium, de 1-octyl-3-méthylimidazolium, de 1-décyl-3-méthylimidazolium, de 1-dodécyl-3-méthylimidazolium, de 1-tétradécyl-3-méthylimidazolium, de 1-hexadécyl-3-méthylimidazolium, de 1-octadécyl-3-méthylimidazolium ; l'hexafluorophosphate de 1-butyl-3-méthylimidazolium, de 1-hexyl-3-méthylimidazolium, de 1-octyl-3-méthylimidazolium ; le tétrafluoroborate de 1-butyl-3-méthylimidazolium, de 1-hexyl-3-méthylimidazolium ; les sels de 1-alkylpyridinium tels que le bromure de 1-éthylpyridinium, de 1-butylpyridinium, de 1-hexylpyridinium, le chlorure de 1-éthylpyridinium, de 1-butylpyridinium ; le chlorure de 1-hexylpyridinium ; l'hexafluorophosphate de 1-butylpyridinium, de 1-hexylpyridinium ; le tétrafluoroborate de 1-butylpyridinium, de 1-hexylpyridinium.

Toutefois, on préfère mettre en oeuvre tout particulièrement les bromure, chlorure, hydrogénosulfate ou hydrogénophosphate de tétrabutylammonium, de méthyltri(n-butyl)ammonium, de N-méthyl-N,N,N-trioctylammonium, de triméthylphénylphosphonium, de tétrabutylphosphonium, de méthyltri(n-butyl)phosphonium, de méthyltri(isobutyl)phosphonium, de diisobutyl-n-octylméthylphosphonium,

Le sel d'onium peut être introduit au cours du procédé de l'invention, à l'état solide ou sous forme d'une solution dans l'un de ses solvants, le plus souvent l'eau.

Le substrat est mis en contact avec le sel d'onium.

La quantité de sel d'onium mise en oeuvre est généralement au moins égale à la quantité stoechiométrique. Ainsi, elle est telle que le rapport molaire entre ledit sel d'onium et le substrat acide varie entre 1 et 5, de préférence, entre 1,2 et 1,5. La borne supérieure n'est pas critique et peut être largement dépassée sans inconvénient car le catalyseur peut être éventuellement recyclé en fin de réaction.

Comme mentionné précédemment, la réaction est conduite, en milieu aqueux, avantageusement en l'absence de tout solvant organique.

Selon un mode préférentiel de réalisation de l'invention, on choisit une concentration en substrat acide aussi élevée que possible selon sa solubilité.

Généralement, la concentration su substrat acide en solution aqueuse varie entre 5 et 40 % en poids et se situe de préférence entre 10 et 20 % en poids.

La réaction est avantageusement effectuée selon le principe "one pot", l'ordre d'introduction des réactifs n'étant pas critique.

La température à laquelle est mis en oeuvre le procédé de l'invention se situe généralement entre 10°C et 60°C, de préférence à température ambiante. Par « température ambiante », on entend une température située le plus souvent entre 15°C et 25°C.

D'un point de vue pratique, on mélange sous agitation, la solution aqueuse du substrat, le sel d'onium qui peut être sous forme liquide ou solide.

Selon une variante du procédé de l'invention, on peut ajouter un solvant organique dans lequel le sel d'onium final est soluble.

Comme exemples, on peut mentionner les hydrocarbures aliphatiques, cycloaliphatiques, aromatiques, halogénés ou non et plus particulièrement, le toluène, le dichlorométhane et le dichlorobenzène.

La quantité de solvant introduite représente généralement la moitié du volume de la phase aqueuse.

On porte le mélange à la température choisie.

En fin de réaction, on obtient un milieu liquide biphasique comprenant une phase organique comprenant le complexe résultant de la réaction du sel de l'acide carboxylique porteur d'au moins un atome d'halogène en position α du groupe carbonyle et du sel d'onium conduisant au déplacement du cation de l'acide carboxylique par l'onium et une phase aqueuse comprenant les différents sels en particulier celui résultant de la réaction du cation de l'acide carboxylique avec l'anion de l'onium.

Le complexe obtenu est présent dans la phase organique qui peut être séparée de la phase aqueuse, notamment par décantation.

On libère la fonction acide du complexe obtenu dans la phase organique par traitement à l'aide d'un acide tel que par exemple, l'acide chlorhydrique, sulfurique, sulfonique ou nitrique.

On met en oeuvre une quantité d'acide allant généralement de la stoechiométrie jusqu'à un excès par exemple de 20 %.

On récupère ledit acide de la phase organique, selon les techniques classiquement utilisées telles que, par exemple, la distillation ou l'extraction à l'aide d'un solvant adéquat comme par exemple un ester, acétate d'éthyle ou de butyle ; un alcool tel que le butanol ou l'octanol ; un hydrocarbure aliphatique halogéné comme le dichlorométhane ou le dichloroéthane.

Un avantage de l'invention est la sélectivité de séparation des espèces fluorées vis-à-vis d'espèces non fluorées.

Le procédé de l'invention permet de traiter des mélanges comprenant des composés mono-, di- ou polyfluorés en particulier des effluents aqueux comprenant lesdits composés fluorés : le composé le plus fluoré étant toujours extrait préférentiellement.

On donne ci-après des exemples de réalisation de l'invention donnés à titre indicatif et sans caractère limitatif.

Dans les exemples, on définit par « RR », le rapport entre le nombre de moles extraites dans la phase organique et le nombre de moles initialement introduites dans la phase aqueuse.

### Exemples 1 à 5

A 10 g d'une solution aqueuse contenant 1,6 g de difluoroacétate de sodium, 0,2 g de trifluoroacétate de sodium et 0,9 g de chlorure de sodium sont ajoutés 22,4 mmol de sel d'onium mentionné dans le tableau (I) soit 1,5 équivalents par rapport à la somme totale de sels de difluoroacétate et de trifluoroacétate.

L'ensemble est laissé sous agitation pendant 30 minutes puis est laissé décanter.

La phase organique supérieure est récupérée par décantation.

Les rendements d'extraction (RR) des sels d'acide difluoroacétique (DFA) et trifluoroacétique (TFA), exprimés par le rapport entre le nombre de moles extraites dans la phase organique et le nombre de moles initialement introduites dans la phase aqueuse, sont résumés dans le tableau suivant :

**Tableau (I)**

| Réf. Ex. | Nature onium | % DFA extrait | % TFA extrait |
|---|---|---|---|
| 1 | Aliquat 336 Chlorure de N-méthyl-N,N,N-trioctylammonium | 64,5 | 99,6 |
| 2 | Bromure de tétrabutylammonium | 65,5 | 99,8 |
| 3 | Chlorure de tétrabutylphosphonium | 36,2 | 97,5 |
| 4 | Chlorure de méthyitributylammonium | 83,6 | 85,5 |
| 5 | Hydrogénosulfate de tétrabutylammonium | 99,5 | 100,0 |

### Exemple 6

A 80 g d'une solution aqueuse contenant 13 g de difluoroacétate de sodium et 1,8 g de trifluoroacétate de sodium, sont ajoutés 67,2 g d'hydrogénosulfate de tétrabutylammonium.

L'ensemble est laissé 10 heures, sous agitation, à température ambiante.

Les phases sont ensuite décantées et séparées.

On obtient 51 g de phase aqueuse et 87 g de phase organique.

L'intégralité des espèces fluorées se trouve en phase organique (RR = 100 %).

Cette phase organique est portée à 74°C sous une pression de 60 mbars jusqu'à la fin de distillation d'eau.

Après retour à pression atmosphérique, 26 g d'acide sulfurique sont ajoutés, et l'ensemble est mis sous pression réduite de 60 mbars et la température est progressivement montée de 54°C jusqu'à 120°C.

On obtient 7,2 g de distillat contenant 5,7 g d'acide difluoroacétique soit un rendement exprimé par le rapport entre le nombre de moles de l'acide difluoroacétique obtenues et le nombre de moles de difluoroacétate de sodium introduites de 54 %.

### Exemple 7

Le trifluoroacétate de potassium (9,7 g; 63,8 mmol), le bromure de tétrabutylammonium (43,4 g; 128 mmol) sont chargés dans un réacteur, en présence d'eau (75 g) et de dichlorométhane (65 g).

L'ensemble est agité à température ambiante et les phases sont décantées.

La phase organique obtenue contient 88 % des anions trifluoroacétate introduits initialement (RR).

## Revendications

1. Procédé de séparation d'un acide carboxylique sous forme salifiée porteur d'au moins un atome d'halogène en position α du groupe carbonyle à partir d'un milieu aqueux le comprenant, **caractérisé par le fait que** l'on met en contact ce dernier avec un sel d'onium conduisant à la formation de deux phases :
- une phase organique comprenant le sel résultant de la réaction du sel de l'acide carboxylique porteur d'au moins un atome d'halogène en position α du groupe carbonyle et du sel d'onium conduisant au déplacement du cation de l'acide carboxylique par l'onium,
- une phase aqueuse comprenant les différents sels en particulier celui résultant de la réaction du cation de l'acide carboxylique avec l'anion de l'onium,
et **par le fait que** l'on sépare ensuite les phases organique et aqueuse et que l'on récupère le sel d'onium de l'acide carboxylique à partir de la phase organique.

2. Procédé selon la revendication 1 **caractérisé par le fait que** le sel de l'acide carboxylique répond à la formule suivante : 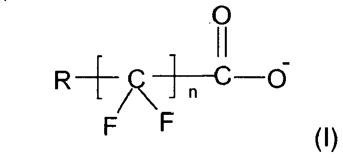 dans ladite formule :
- n est un nombre compris entre 0 et 10,
- si n est égal à 0, R représente un groupe R₁R₂CF de formule (II) dans lequel R₁ et R₂ représentent un atome d'hydrogène, un atome de fluor, chlore ou brome,
- si n est différent de 0, R représente un atome d'hydrogène, un atome de fluor, chlore ou brome ou une fonction carboxylate.

3. Procédé selon la revendication 2 **caractérisé par le fait que** l'on met en oeuvre un sel des acides carboxyliques suivants, seul ou en mélange :
- l'acide fluoroacétique,
- l'acide difluoroacétique, DFA
- l'acide trifluoroacétique, TFA
- l'acide chlorodifluoroacétique, CDFA
- l'acide bromodifluoroacétique,
- l'acide perfluoropropanoïque,
- l'acide perfluorobutanoïque,
- l'acide perfluoroheptanoïque,
- l'acide perfluorooctanoïque
- l'acide perfluorodécanoïque,
- l'acide tétrafluorosuccinique,
- l'acide hexafluoroglutarique.

4. Procédé selon l'une des revendications 1 à 3 **caractérisé par le fait que** le sel de l'acide carboxylique est un sel de métal alcalin, de préférence le sodium ou le potassium

5. Procédé selon la revendication 1 **caractérisé par le fait que** le sel de l'acide carboxylique est un sel de métal alcalin du DFA, TFA ou de leurs mélanges.

6. Procédé selon l'une des revendications 1 à 5 **caractérisé par le fait que** le sel d'onium comprend un onium qui répond à l'une des formules générales suivantes : 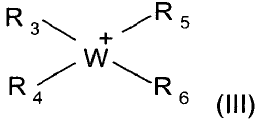 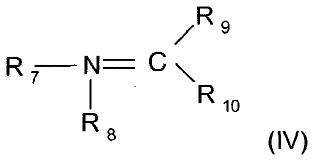 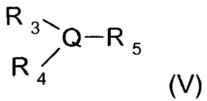 dans lesdites formules :
- W représente N ou P,
- Q représente S, S=O ou C,
- R₃, R₄, R₅ et R₆, identiques ou différents représentent :
. un groupe alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
. un groupe alcényle, linéaire ou ramifié, ayant 2 à 12 atomes de carbone ;
. un groupe aryle ayant 6 à 10 atomes de carbone, éventuellement substitué par un ou plusieurs groupements ou atomes alkyle ayant 1 à 4 atomes de carbone, alkoxy, alkoxycarbonyle, le groupe alkoxy ayant 1 à 4 atomes de carbone, ou halogène ;
. deux desdits groupes R₃ à R₆ pouvant former ensemble un groupe alkylène, alcénylène ou alcadiènylène, linéaire ou ramifié ayant de 3 à 6 atomes de carbone ;
- R₇, R₈, R₉, R₁₀ sont identiques ou différents et représentent :
. un atome d'hydrogène,
. un groupe alkyle linéaire ou ramifié contenant de 1 à 6 atomes de carbone ;
. les groupes R₉ et R₁₀ pouvant former ensemble un groupe alkylène contenant de 3 à 6 atomes de carbone ;
. les groupes R₈ et R₉ ou R₈ et R₁₀ pouvant former ensemble un groupe alkylène, alcénylène ou alcadiènylène, contenant 3 ou 4 atomes de carbone et constituant avec l'atome d'azote un hétérocycle azoté éventuellement substitué et éventuellement l'un des atomes de carbone pouvant être remplacé par un atome d'azote éventuellement porteur d'un groupe R₁₁ qui est un groupe alkyle linéaire ou ramifié contenant de 1 à 20 atomes de carbone.

7. Procédé selon l'une des revendications 1 à 6 **caractérisé par le fait que** l'onium est un ion ammonium, phosphonium, imidazolinium ou pyridinium.

8. Procédé selon l'une des revendications 1 à 7 **caractérisé par le fait que** l'anion du sel d'onium peut être choisi parmi les anions suivants : sulfate, hydrogénosulfate, hydrogénophosphate, bromure, chlorure.

9. Procédé selon l'une des revendications 1 à 8 **caractérisé par le fait que** le sel d'onium est un bromure, chlorure, hydrogénosulfate ou hydrogénophosphate de tétrabutylammonium, de méthyltri(n-butyl)ammonium, de N-méthyl-N,N,N-trioctylammonium, de triméthylphénylphosphonium, de tétrabutylphosphonium, de méthyltri(n-butyl)phosphonium, de méthyltri(isobutyl)phosphonium, de diisobutyl-n-octylméthylphosphonium.

10. Procédé selon l'une des revendications 1 à 9 **caractérisé par le fait que** la quantité de sel d'onium mise en oeuvre est telle que le rapport molaire entre ledit sel d'onium et le substrat acide varie entre 1 et 5, de préférence, entre 1,2 et 1,5.

11. Procédé selon l'une des revendications 1 à 10 **caractérisé par le fait que** la température à laquelle est mis en oeuvre le procédé de l'invention se situe entre 10°C et 60°C, de préférence à température ambiante.

12. Procédé selon l'une des revendications 1 à 11 **caractérisé par le fait que** l'on mélange sous agitation, la solution aqueuse du substrat, le sel d'onium qui peut être sous forme liquide ou solide.

13. Procédé selon l'une des revendications 1 à 12 **caractérisé par le fait que** l'on peut ajouter un solvant organique dans lequel le sel d'onium final est soluble.

14. Procédé selon l'une des revendications 1 à 13 **caractérisé par le fait que** le complexe obtenu est présent dans la phase organique qui peut être séparée de la phase aqueuse, de préférence par décantation.

15. Procédé selon l'une des revendications 1 à 14 **caractérisé par le fait que** l'on libère la fonction acide du complexe obtenu dans la phase organique, par traitement à l'aide d'un acide de préférence, l'acide chlorhydrique, sulfurique, sulfonique ou nitrique.

16. Procédé selon l'une des revendications 1 à 15 **caractérisé par le fait que** l'on récupère ledit acide de la phase organique par distillation ou extraction à l'aide d'un solvant adéquat.

17. Application du procédé décrit dans l'une des revendications 1 à 16 pour le traitement des mélanges comprenant des composés mono-, di- ou polyfluorés en particulier des effluents aqueux comprenant lesdits composés fluorés.

## Patentansprüche

1. Verfahren zur Abtrennung einer Carbonsäure in versalzter Form, die mindestens ein Halogenatom in α-Stellung zur Carbonylgruppe trägt, aus einem diese umfassenden wässrigen Medium, **dadurch gekennzeichnet, dass** man letzteres mit einem Oniumsalz in Berührung bringt, was zur Bildung von zwei Phasen führt:
- einer organischen Phase, umfassend das sich aus der Reaktion des Carbonsäuresalzes, das mindestens ein Halogenatom in α-Stellung zur Carbonylgruppe trägt, und des Oniumsalzes, die zum Ersatz des Kations der Carbonsäure durch das Onium führt, ergebende Salz,
- eine wässrige Phase, umfassend die verschiedenen Salze, insbesondere dasjenige, das sich aus der Reaktion des Kations der Carbonsäure mit dem Anion des Oniums ergibt,
und dadurch, dass man dann die organische Phase und die wässrige Phase trennt und das Oniumsalz der Carbonsäure aus der organischen Phase gewinnt.

2. Verfahren nach Anspruch 1, **dadurch gekennzeichnet, dass** das Carbonsäuresalz der folgenden Formel entspricht: 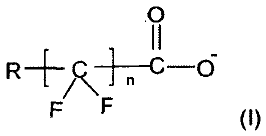 worin:
- n für eine Zahl zwischen 0 und 10 steht,
- dann, wenn n gleich 0 ist, R für eine Gruppe R₁R₂CF der Formel (II), worin R₁ und R₂ für ein Wasserstoffatom, ein Fluor-, Chlor- oder Bromatom stehen, steht,
- dann, wenn n von 0 verschieden ist, R für ein Wasserstoffatom, ein Fluor-, Chlor- oder Bromatom oder eine Carboxylatfunktion steht.

3. Verfahren nach Anspruch 2, **dadurch gekennzeichnet, dass** man ein Salz der folgenden Carbonsäuren alleine oder als Gemisch einsetzt:
- Fluoressigsäure,
- Difluoressigsäure, DFA
- Trifluoressigsäure, TFA
- Chlordifluoressigsäure, CDFA
- Bromdifluoressigsäure,
- Perfluorpropansäure,
- Perfluorbutansäure,
- Perfluorheptansäure,
- Perfluoroctansäure,
- Perfluordecansäure,
- Tetrafluorbernsteinsäure,
- Hexafluorglutarsäure.

4. Verfahren nach einem der Ansprüche 1 bis 3, **dadurch gekennzeichnet, dass** es sich bei dem Carbonsäuresalz um ein Alkalimetallsalz, vorzugsweise ein Natrium- oder Kaliumsalz, handelt.

5. Verfahren nach Anspruch 1, **dadurch gekennzeichnet, dass** es sich bei dem Carbonsäuresalz um ein Alkalimetallsalz von DFA, TFA oder Gemischen davon handelt.

6. Verfahren nach einem der Ansprüche 1 bis 5, **dadurch gekennzeichnet, dass** das Oniumsalz ein Onium umfasst, das einer der folgenden allgemeinen Formeln entspricht: 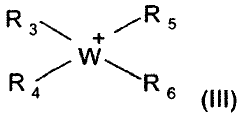 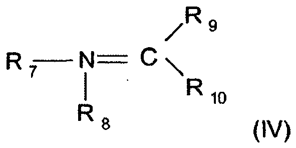 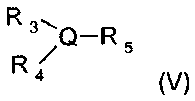 worin:
- W für N oder P steht,
- Q für S, S=O oder C steht,
- R₃, R₄, R₅ und R₆ gleich oder verschieden sind und die folgende Bedeutung besitzen:
. eine lineare oder verzweigte Alkylgruppe, die 1 bis 16 Kohlenstoffatome aufweist und gegebenenfalls durch eine oder mehrere der folgenden Gruppen bzw. ein oder mehrere der folgenden Atome substituiert ist: Phenyl, Hydroxyl, Halogen, Nitro, Alkoxy oder Alkoxycarbonyl, wobei die Alkoxygruppen 1 bis 4 Kohlenstoffatome aufweisen;
. eine lineare oder verzweigte Alkenylgruppe mit 2 bis 12 Kohlenstoffatomen;
. eine Arylgruppe mit 6 bis 10 Kohlenstoffatomen, die gegebenenfalls durch eine oder mehrere der folgenden Gruppen bzw. ein oder mehrere der folgenden Atome substituiert ist: Alkyl mit 1 bis 4 Kohlenstoffatomen, Alkoxy, Alkoxycarbonyl, wobei die Alkoxygruppe 1 bis 4 Kohlenstoffatome aufweist, oder Halogen;
. wobei zwei Gruppen R₃ bis R₆ zusammen eine lineare oder verzweigte Alkylen-, Alkenylen- oder Alkadienylengruppe mit 3 bis 6 Kohlenstoffatomen bilden können;
- R₇, R₈, R₉ und R₁₀ gleich oder verschieden sind und die folgende Bedeutung besitzen:
. ein Wasserstoffatom,
. eine lineare oder verzweigte Alkylgruppe mit 1 bis 6 Kohlenstoffatomen;
. wobei die Gruppen R₉ und R₁₀ zusammen eine Alkylengruppe mit 3 bis 6 Kohlenstoffatomen bilden können;
. wobei die Gruppen R₈ und R₉ oder R₈ und R₁₀ zusammen eine Alkylen-, Alkenylen- oder Alkadienylengruppe, die 3 oder 4 Kohlenstoffatome aufweist und mit dem Stickstoffatom einen gegebenenfalls substituierten Stickstoffheterocyclus bildet, bilden können, wobei eines der Kohlenstoffatome gegebenenfalls durch ein Stickstoffatom, das gegebenenfalls eine Gruppe R₁₁ trägt, bei der es sich um eine lineare oder verzweigte Alkylgruppe mit 1 bis 20 Kohlenstoffatomen handelt, ersetzt sein kann.

7. Verfahren nach einem der Ansprüche 1 bis 6, **dadurch gekennzeichnet, dass** es sich bei dem Onium um ein Ammonium-, Phosphonium-, Imidazolinium- oder Pyridiniumion handelt.

8. Verfahren nach einem der Ansprüche 1 bis 7, **dadurch gekennzeichnet, dass** das Anion des Oniumsalzes aus den folgenden Anionen ausgewählt sein kann: Sulfat, Hydrogensulfat, Hydrogenphosphat, Bromid, Chlorid.

9. Verfahren nach einem der Ansprüche 1 bis 8, **dadurch gekennzeichnet, dass** es sich bei dem Oniumsalz um ein Bromid, Chlorid, Hydrogensulfat oder Hydrogenphosphat von Tetrabutylammonium, Methyltri(n-butyl)ammonium, N-Methyl-N,N,N-tri-octylammonium, Trimethylphenylphosphonium, Tetrabutylphosphonium, Methyltri(n-butyl)phosphonium, Methyltri(isobutyl)phosphonium oder Diisobutyl-n-octylmethylphosphonium handelt.

10. Verfahren nach einem der Ansprüche 1 bis 9, **dadurch gekennzeichnet, dass** die verwendete Oniumsalzmenge derart beschaffen ist, dass das Molverhältnis zwischen dem Oniumsalz und dem Säuresubstrat zwischen 1 und 5 und vorzugsweise zwischen 1,2 und 1,5 variiert.

11. Verfahren nach einem der Ansprüche 1 bis 10, **dadurch gekennzeichnet, dass** die Temperatur, bei der das erfindungsgemäße Verfahren durchgeführt wird, zwischen 10°C und 60°C und vorzugsweise bei Umgebungstemperatur liegt.

12. Verfahren nach einem der Ansprüche 1 bis 11, **dadurch gekennzeichnet, dass** man die wässrige Lösung des Substrats und das Oniumsalz, das in flüssiger oder fester Form vorliegen kann, unter Rühren mischt.

13. Verfahren nach einem der Ansprüche 1 bis 12, **dadurch gekennzeichnet, dass** man ein organisches Lösungsmittel zusetzt, in dem das letztendliche Oniumsalz löslich ist.

14. Verfahren nach einem der Ansprüche 1 bis 13, **dadurch gekennzeichnet, dass** der erhaltene Komplex in der organischen Phase vorliegt, welche von der wässrigen Phase getrennt werden kann, vorzugsweise durch Dekantieren.

15. Verfahren nach einem der Ansprüche 1 bis 14, **dadurch gekennzeichnet, dass** man die Säurefunktion des in der organischen Phase erhaltenen Komplexes durch Behandlung mit einer Säure, vorzugsweise Salzsäure, Schwefelsäure, Sulfonsäure oder Salpetersäure, freisetzt.

16. Verfahren nach einem der Ansprüche 1 bis 15, **dadurch gekennzeichnet, dass** man die Säure aus der organischen Phase durch Destillation oder Extraktion mit einem geeigneten Lösungsmittel gewinnt.

17. Anwendung des in einem der Ansprüche 1 bis 16 beschriebenen Verfahrens zur Behandlung von Gemischen, die mono-, di- oder polyfluorierte Verbindungen enthalten, insbesondere wässrigen Austragsströmen, die diese fluorierten Verbindungen enthalten.

## Claims

1. Process for separating a carboxylic acid in salified form bearing at least one halogen atom at the α position of the carbonyl group from an aqueous medium comprising it, **characterized by** the fact that the latter is brought into contact with an onium salt leading to the formation of two phases:
- an organic phase comprising the salt resulting from the reaction of the carboxylic acid salt bearing at least one halogen atom at the α position of the carbonyl group and of the onium salt leading to the displacement of the cation of the carboxylic acid by the onium,
- an aqueous phase comprising the various salts in particular the one resulting from the reaction of the cation of the carboxylic acid with the anion of the onium,
and by the fact that the organic and aqueous phases are then separated and the onium salt of the carboxylic acid is recovered from the organic phase.

2. Process according to Claim 1, **characterized by** the fact that the carboxylic acid salt corresponds to the following formula: 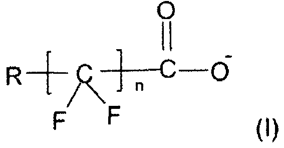 in said formula:
- n is a number between 0 and 10,
- if n is equal to 0, R represents an R₁R₂CF group of formula (II) in which R₁ and R₂ represent a hydrogen atom, or a fluorine, chlorine or bromine atom,
- if n is different from 0, R represents a hydrogen atom, a fluorine, chlorine or bromine atom or a carboxylate function.

3. Process according to Claim 2, **characterized by** the fact that use is made of a salt of the following carboxylic acids, alone or as a mixture:
- fluoroacetic acid,
- difluoroacetic acid, DFA,
- trifluoroacetic acid, TFA,
- chlorodifluoroacetic acid, CDFA,
- bromodifluoroacetic acid,
- perfluoropropanoic acid,
- perfluorobutanoic acid,
- perfluoroheptanoic acid,
- perfluorooctanoic acid,
- perfluorodecanoic acid,
- tetrafluorosuccinic acid,
- hexafluoroglutaric acid.

4. Process according to one of Claims 1 to 3, **characterized by** the fact that the salt of the carboxylic acid is an alkali metal salt, preferably the sodium or potassium salt.

5. Process according to Claim 1, **characterized by** the fact that the carboxylic acid salt is an alkali metal salt of DFA, TFA or mixtures thereof.

6. Process according to one of Claims 1 to 5, **characterized by** the fact that the onium salt comprises an onium which corresponds to one of the following general formulae: 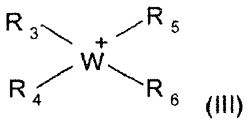 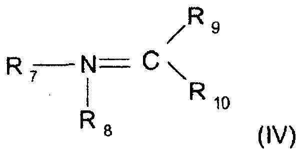 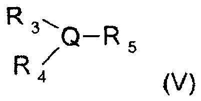 in said formulae:
- W represents N or P,
- Q represents S, S=O or C,
- R₃, R₄, R₅ and R₆, which are identical or different, represent:
. a linear or branched alkyl group having 1 to 16 carbon atoms and optionally substituted with one or more of the following groups or atoms: phenyl, hydroxyl, halogen, nitro, alkoxy or alkoxycarbonyl, the alkoxy groups having 1 to 4 carbon atoms;
. a linear or branched alkenyl group having 2 to 12 carbon atoms;
. an aryl group having 6 to 10 carbon atoms, optionally substituted with one or more of the following groups or atoms: alkyl having 1 to 4 carbon atoms, alkoxy, alkoxycarbonyl, the alkoxy group having 1 to 4 carbon atoms, or halogen;
. two of said R₃ to R₆ groups possibly together forming a linear or branched alkylene, alkenylene or alkadienylene group having 3 to 6 carbon atoms;
- R₇, R₈, R₉, R₁₀ are identical or different and represent:
. a hydrogen atom,
. a linear or branched alkyl group containing from 1 to 6 carbon atoms;
. the R₉ and R₁₀ groups possibly together forming an alkylene group containing from 3 to 6 carbon atoms;
. the R₈ and R₉ or R₈ and R₁₀ groups possibly together forming an alkylene, alkenylene or alkadienylene group containing 3 or 4 carbon atoms and constituting, with the nitrogen atom, an optionally substituted nitrogen-containing heterocycle and it being possible for one of the carbon atoms to optionally be replaced with a nitrogen atom optionally bearing an R₁₁ group which is a linear or branched alkyl group containing from 1 to 20 carbon atoms.

7. Process according to one of Claims 1 to 6, **characterized by** the fact that the onium is an ammonium, phosphonium, imidazolinium or pyridinium ion.

8. Process according to one of Claims 1 to 7, **characterized by** the fact that the anion of the onium salt may be chosen from the following anions: sulfate, hydrogensulfate, hydrogenphosphate, bromide, chloride.

9. Process according to one of Claims 1 to 8, **characterized by** the fact that the onium salt is a bromide, chloride, hydrogensulfate or hydrogenphosphate of tetrabutylammonium, methyltri(n-butyl)ammonium, N-methyl-N,N,N-trioctylammonium, trimethylphenylphosphonium, tetrabutylphosphonium, methyltri(n-butyl)-phosphonium, methyltri(isobutyl)phosphonium or diisobutyl-n-octylmethylphosphonium.

10. Process according to one of Claims 1 to 9, **characterized by** the fact that the amount of onium salt used is such that the molar ratio between said onium salt and the acid substrate varies between 1 and 5, preferably between 1.2 and 1.5.

11. Process according to one of Claims 1 to 10, **characterized by** the fact that the temperature at which the process of the invention is carried out is between 10°C and 60°C, preferably at ambient temperature.

12. Process according to one of Claims 1 to 11, **characterized by** the fact that mixed, with stirring, are the aqueous solution of the substrate, and the onium salt which may be in liquid or solid form.

13. Process according to one of Claims 1 to 12, **characterized by** the fact that an organic solvent in which the final onium salt is soluble may be added.

14. Process according to one of Claims 1 to 13, **characterized by** the fact that the complex obtained is present in the organic phase which may be separated from the aqueous phase, preferably by decantation.

15. Process according to one of Claims 1 to 14, **characterized by** the fact that the acid function of the complex obtained in the organic phase is released by treatment using an acid, preferably hydrochloric, sulfuric, sulfonic or nitric acid.

16. Process according to one of Claims 1 to 15, **characterized by** the fact that said acid is recovered from the organic phase by distillation or extraction using a suitable solvent.

17. Application of the process described in one of Claims 1 to 16 for the treatment of mixtures comprising monofluorinated, difluorinated or polyfluorinated compounds, in particular aqueous effluents comprising said fluorinated compounds.
